# EUROPEAN PATENT SPECIFICATION

(11) **EP 1 289 489 B1**
(45) Date of publication and mention of the grant of the patent: **05.12.2007**
(21) Application number: 01945236.6
(22) Date of filing: 01.06.2001
(51) Int. Cl.: A61Q 19/02, A61K 8/60, A61K 8/36

(54) **TOPICAL AGENT FOR DERMATOLOGICAL USE CONTAINING 4-HYDROXYPHENYL-ALPHA-D-GLUCOPYRANOSIDE**
TOPISCHES MITTEL ZUR DERMATOLOGISCHEN VERWENDUNG ENTHALTEND 4-HYDROXYPHENYL-ALPHA-D-GLUCOPYRANOSID
AGENT TOPIQUE POUR UTILISATION DERMATOLOGIQUE CONTENANT DU 4-HYDROXYPHENYL-ALPHA-D-GLUCOPYRANOSIDE

(30) Priority: 02.06.2000 JP 2000165590
(43) Date of publication of application: 12.03.2003
(60) Divisional application: 07010600.0
(73) Proprietor: PENTAPHARM Ltd., 4002 Basel (CH); Ezaki Glico Co., Ltd., Osaka-shi, Osaka 555-8502 (JP)
(72) Inventor: KURIKI, Takashi, Suita-shi, Osaka (JP); NAKAE, Takashi, Sanda-shi, Hyogo (JP); NISHIMURA, Takahisa, Saidaiji, Nara-shi, Nara (JP); NAKAYAMA, Hiroki, Shinjuku-ku, Tokyo (JP)
(74) Representative: Braun, André jr.
(86) International application number: PCT/EP2001/006281
(87) International publication number: WO 2001/091715

(56) References cited:
- EP-A- 1 153 602
- WO-A-01/60324
- DATABASE CAPLUS [Online] CHEMICAL ABSTRACTS SERVICE, COLUMBUS, OHIO, US; retrieved from STN Database accession no. 1997:649706 XP002193686
- DATABASE CAPLUS [Online] CHEMICAL ABSTRACTS SERVICE, COLUMBUS, OHIO, US; retrieved from STN Database accession no. 1995:275688 XP002193687 & JP 06 284896 A (KURASHIKI BOSEKI KK) 11 October 1994 (1994-10-11)
- DATABASE CAPLUS [Online] CHEMICAL ABSTRACTS SERVICE, COLUMBUS, OHIO, US; retrieved from STN Database accession no. 1995:846101 XP002193688
- DATABASE CAPLUS [Online] CHEMICAL ABSTRACTS SERVICE, COLUMBUS, OHIO, US; retrieved from STN Database accession no. 1994:577883 XP002193689 & JP 06 153976 A (KIKKOMAN) 3 June 1994 (1994-06-03)
- DATABASE CAPLUS [Online] CHEMICAL ABSTRACTS SERVICE, COLUMBUS, OHIO, US; retrieved from STN Database accession no. 1993:175535 XP002193690 & JP 05 000932 A (KURASHIKI SPINNING CO) 8 January 1993 (1993-01-08)
- DATABASE REGISTRY [Online] retrieved from STN Database accession no. 84380-01-8/rn XP002193691

## Description

### Art to which the invention pertains

The present invention is related to a topical agent for dermatological use which whiten the skin color or prevent its blackening and prevent or relieve liver spots, freckles, and which show generally desirable formulation properties in terms of the safety and stability.

### Conventional technology

Various melanin formation preventing agents have been used to whiten the skin color or prevent its blackening and prevent or relieve skin troubles such as liver spots and freckles caused due to excessive exposure to UV rays. These agents include 1,4-dihydroxybenzene, β-arbutin, vitamin C and its derivatives, and kojic acid.

However, vitamin C, 1,4-dihydroxybenzene and kojic acid are extremely unstable with respect to heat and oxidation in water. When added to topical agents for dermatological use, these compounds decompose over time and cause coloration. Their derivatives, such as phosphate-ascorbyl magnesium and β-arbutin, which is obtained as a result of the β-binding of glucose to one of the hydroxy groups of 1,4-dihydroxybenzene, are not necessarily satisfactory in terms of efficacy although they are more stable than their parent compounds with respect to heat and oxidation.

To overcome this problem, various inventions have been made and applications for patents have been submitted, such as cosmetics which contain, in addition to β-arbutin, ingredients with skin whitening effects, such as UV absorbents, anti-inflammatory agents, and placenta extracts, so that synergistic effects can be obtained (e.g., Toku-kai-hei 5-186324 Patent Gazette) and topical agents for dermatological use which contain pantethine-S-sulfonic acid or its salt and which prevent decomposition and coloration over time (Toku-kai-hei 5-58926 Patent Gazette). However, these substances cannot be added to cosmetic products in a quantity large enough to obtain clinically significant synergistic effects due to problems related to safety and bad feeling experienced upon contact with the skin.

The authors of the present invention looked for substances which are stable when used in topical agents for dermatological use and which are safer and more effective than conventional compounds. In this effort, they discovered 4-hydroxyphenyl-α-D-glucopyranoside as a substance which satisfies these requirements and have applied for a patent (Tokugan 2000-43366). However, its efficacy had to be further improved in order to expect clinically significant whitening effects.

A patent has been disclosed for amylase X-23 as an enzyme which transfers sugars to the phenol group of 1,4-dihydroxybenzene through α-binding (Patent No. 2662667).

### Problems which the present invention is designed to solve

As mentioned above, various attempts have been made to enhance skin whitening effects, but none has been found satisfactory. The present invention uses 4-hydroxyphenyl-α-D-glucopyranoside, which shows marked skin whitening effects even when used alone, and add ascorbic acid which enhances or supplements its effects to produce a topical agent with dermatological use which is more effective than conventional products.

### Methods used to solve the problems

The authors of the present invention conducted studies to solve the above-mentioned problems, found that the combination of 4-hydroxyphenyl-α-D-glucopyranoside and ascorbic acid results in a marked enhancement of the effects of 4-hydroxyphenyl-α-D-glucopyranoside, which shows far greater skin whitening effects than conventional products even when used alone, and completed the present invention.

The present invention pertains to a topical agent for dermatological use which is characterized by the fact that it contains 4-hydroxyphenyl-α-D-glucopyranoside and ascorbic acid. It also pertains to a topical agent for dermatological use described above which is characterized by the fact that 4-hydroxyphenyl-α-D-glucopyranoside is obtained using α-amylase, as well as those in which α-amylase is amylase X-23.

### Working of the invention

When ascorbic acid is added to a topical agent for dermatological use in combination with 4-hydroxyphenyl-α-D-glucopyranoside, it increases the whitening effects or stability of 4-hydroxyphenyl-α-D-glucopyranoside.

Among various types of ascorbic acid, L-ascorbic acid, generally called vitamin C, promotes cell respiration, enzymatic activation, and collagen formation due to its strong reducing effects and also reduces melanin.

Efficacy is seen when ascorbic acid is added at 0.01 w/w% or more in the topical agent for dermatological use. The upper limit of content is about 10%.

The ascorbic acid enhances the whitening effects of the topical agent for dermatological use under the present invention when concurrently used with 4-hydroxyphenyl-α-D-glucopyranoside.

The ascorbic acid does not affect 4-hydroxyphenyl-α-D-glucopyranoside in the topical agent when used with 4-hydroxyphenyl-α-D-glucopyranoside in the indicated range of the contents. It remains stable for a long period of time and shows excellent whitening effects. It content may be increased or decreased depending on the degree of expected effects.

4-hydroxyphenyl-α-D-glucopyranoside used in the present invention is explained below in great detail.

4-hydroxyphenyl-α-D-glucopyranoside is obtained as a result of α-binding of D-glucose with the phenol group of 1,4-dihydroxybenzene. β-Arbutin, which is obtained as a result of β-binding of D-glucose with the phenol group of 1,4-dihydroxybenzene, is commonly used in topical agents for dermatological use because of its skin whitening effects. 4-Hydroxyphenyl-α-D-glucopyranoside is not only more effective than β-arbutin but is also more stable and safer when applied to the skin.

The content of 4-hydroxyphenyl-α-D-glucopyranoside in the topical agent for dermatological use should be 0.01-30 w/w%, preferably 0.05-20 w/w% and more preferably 0.1-10 w/w%.

4-Hydroxyphenyl-α-D-glucopyranoside can be chemically manufactured, but it can also be obtained by converting 1,4-dihydroxybenzene into glycoside using enzymes deriving from bacteria. For example, this can be achieved using sucrose as a sugar donor and *Leuconostoc mesenterioides-*derived sucrose phosphorylase as an enzyme.

4-Hydroxyphenyl-α-D-glucopyranoside can also be obtained using soluble starch as a sugar donor and Bacillus *subtilis*-derived α-amylase as an enzyme. In this method, the use of amylase X-23 makes it possible to efficiently obtain 4-hydroxyphenyl-α-D-glucopyranoside on an industrial scale.

How 4-hydroxyphenyl-α-D-glucopyranoside used in the present invention can be obtained using amylase X-23, a type of amylase obtained from *Bacillus subtilis*, is outlined below. Amylase X-23 decomposes glucan with α-1,4 bond in the presence of both phenol related compounds and glucan with α-1,4-bond, and transfers sugar to the OH group of phenol related compounds by α-binding. The optimal pH for sugar transfer ranges between 5 and 8. Sugar transfer can be achieved in a stable manner at pH 5.5 and 30-70°C. Refer to Patent No. 2662667 Patent Gazette for greater detail.

Other ingredients commonly used for the production of topical agents for dermatological use, including cosmetics and drugs, can be added, if necessary, to the topical agent for dermatological use under the present invention. These ingredients include oil, antioxidants, surface active detergents, moisturizing agents, moistening agents, aromatics, water, alcohol, viscous agents, antiseptics, coloring agents, powder, drugs, chelating agents, and pH adjusting agents. These ingredients must be added in amounts which do not qualitatively or quantitatively affect the quality of the topical agent for dermatological use covered by the present invention.

The topical agent for dermatological use covered by the present invention may be made available in any dosage form, including solutions such as toilet lotion, emulsified preparations such as milky liquid and cream, ointment, viscous gel, dispersion, and powder.

When manufacturing the topical agent for dermatological use covered by the present invention in the form of lotion, emulsion and viscous gel, greater efficacy can be obtained by combining the following water-soluble viscous agents with lower alcohol such as ethanol and isopropanol: plant-derived macromolecules (such as gum arabic, tragacanth gum, galactan, Cyamoposis gum, carrageenan, pectin, quince seed (marmelo) extract and brown algae powder), microorganism-derived macromolecules (such as xanthan gum, dextran and pullulan), animal-derived macromolecules (such as collagen, casein, albumin and gelatin), starch (such as carboxymethyl starch and methylhydroxy starch), cellulose (such as methylcellulose, nitrocellulose, ethylcellulose, methylhydroxypropylcellulose, hydroxyethylcellulose, hydroxypropylcellulose, carboxymethylcellulose, crystalline cellulose, cellulose powder), vinyl macromolecules (polyvinyl alcohol, polyvinylmethylether, polyvinylpyrolidone and carboxyvinyl-polymer), acryl macromolecules (such as polyacrylic acid and its salts and polyacrylimide), organic viscous agents (such as glycyrrhizic acid and alginic acid), and inorganic viscous agents (such as bentonite, hectolite, labonite, aluminum silicate magnesium and silicic anhydride).

The content of water-soluble viscous agents in the topical agent for dermatological use should be 0.01-5 w/w%, preferably 0.1-3 w/w%, while the content of lower alcohol in topical agents for dermatological use should be 0.3-35 w/w%. It is desirable to adjust the ratio of 4-hydroxyphenyl-α-D-glucopyranoside and lower alcohol (weight) to 3:1 to 1:3.

### Working example

4-Hydroxyphenyl-α-D-glucopyranoside used in all examples shown below is obtained by allowing amylase X-23 to act in the presence of 1,4-dihydroxybenzene and maltopentaose. All contents shown below are based on w/w%.

### Example 1: pack

A pack product was prepared by the conventional method using the formula shown below.

| | |
|---|---|
| Polyvinyl alcohol | 16.0 |
| Polyethylene glycol | 4.0 |
| Propylene glycol | 7.0 |
| Ethanol | 11.0 |
| Methylparaben | 0.1 |
| 4-Hydroxyphenyl-α-D-glucopyranoside | 7.0 |
| Dihydroxy aluminum allantoinate | 3.0 |
| Ascorbic acid | 1.0 |
| Nordihydroguaiaretic acid | 5.0 |
| Citric acid | 0.3 |
| Sodium citrate | 0.7 |
| Flavor | 0.1 |
| Ion exchanged water | Remainder |

The topical agent for dermatological use obtained in Example 1 showed good skin whitening effects with only slight skin irritation and sensitization potential. It also showed good stability over time.

### Effects of the invention

The topical agent for dermatological use covered by the present invention enhance the effects of 4-hydroxyphenyl-α-D-glucopyranoside due to synergistic effects because it combine 4-hydroxyphenyl-α-D-glucopyranoside, which is safe and stable and exerts good skin whitening effects even when used alone, and ascorbic acid. Therefore, the topical agent for dermatological use covered by the present invention shows marked skin whitening effects and blackening prevention effects and effectively prevents and relieves liver spots and freckles. It was also shown to be safe and stable and is extremely useful as cosmetics and therapeutic agents.

## Claims

1. A topical agent for dermatological use comprising 4-hydroxyphenyl-α-D-glucopyranoside and ascorbic acid.

2. A topical agent according to claim 1 wherein the 4-hydroxyphenyl-α-D-glucopyranoside is obtained using α-amylase.

3. A topical agent according to claim 2 wherein the α-amylase is amylase X-23.

## Patentansprüche

1. Topischer Wirkstoff zur dermatologischen Anwendung enthaltend 4-Hydroxyphenyl-α-D-glucopyranosid und Ascorbinsäure.

2. Topischer Wirkstoff nach Anspruch 1, **dadurch gekennzeichnet, dass** 4-Hydroxyphenyl-α-D-glucopyranosid unter Verwendung von α-Amylase hergestellt wird.

3. Topischer Wirkstoff nach Anspruch 2, worin die α-Amylase Amylase X-23 ist.

## Revendications

1. Principe actif topique à usage dermatologique contenant du 4-hydroxyphényl-α-D-glucopyranoside et de l'acide ascorbique.

2. Principe actif topique selon la revendication 1, **caractérisé en ce que** le 4-hydroxyphényl-α-D-glucopyranoside est obtenu en utilisant de l'α-amylase.

3. Principe actif topique selon la revendication 2, **caractérisé en ce que** l'-α amylase est l'amylase X-23.
